# EUROPEAN PATENT SPECIFICATION

(11) **EP 1 071 382 B1**
(45) Date of publication and mention of the grant of the patent: **25.07.2007**
(21) Application number: 99916069.0
(22) Date of filing: 16.04.1999
(51) Int. Cl.: A61F 2/20

(54) **VOICE-PRODUCING PROSTHESIS**
STIMMPROTHESE
PROTHESE VOCALE

(30) Priority: 17.04.1998 NL 1008917
(43) Date of publication of application: 31.01.2001
(73) Proprietor: Adeva Medical GmbH, 23556 Lübeck (DE)
(72) Inventor: DE VRIES, Marinus, Pieter, NL-9831 PR Aduard (NL); VAN DER PLAATS, Arjan, NL-9712 JR Groningen (NL); SCHUTTE, Harm, Kornelis, NL-9745 DM Groningen (NL); VERKERKE, Gijsbertus, Jacob, NL-9753 EP Haren (NL)
(74) Representative: Prins, Adrianus Willem
(86) International application number: PCT/NL1999/000221
(87) International publication number: WO 1999/053868

(56) References cited:
- EP-A- 0 651 980
- DE-A- 2 253 496
- NL-C- 1 003 509
- US-A- 4 223 411

## Description

As part of the treatment of cancer of the throat it often occurs that the larynx, including the vocal folds and the epiglottis and, if required, part of the pharynx, is removed (laryngectomy).

To enable the patient to eat and drink thereafter without foods or drinks landing in the trachea which is no longer closable now, the trachea is passed outwards so that a stoma is obtained in the throat (a tracheostoma).

To also enable voice production again, it is usual to place a valve in the wall between the esophagus and the trachea. Air flowing out of the lungs can then be passed from the trachea through the valve to the upper part of the esophagus of which tissue along which the air flows is vibrated by the passing air to generate a voice or at least a sound which has to perform this function. The patient can then speak by keeping the tracheostoma closed so that air is passed through the valve to the esophagus.

Similar devices are disclosed in documents NL-C-1003509 and EP-A-0651980.

Drawbacks of this solution are that the voice sounds unnaturally "belching", that speech of such patients is hard to understand, and that the base frequency of the generated sound is too low (40-70 Hz vs. 90-120 Hz for healthy men and 190-220 Hz for healthy women).

Many efforts have meanwhile been made to solve these problems, inter alia by providing voice-producing prostheses in electronic form or in a form driven by exhaled air.

Known electronic prostheses are provided with a voltage source and a manual control outside the body. The vibration source is then pressed against the throat or incorporated in a dental prosthesis. Important drawbacks of such apparatuses are the difficulty of learning the above manual control and the robot-like voice.

Another voice-producing prosthesis already known is arranged to be driven by air. This prosthesis may be connected to the tracheostoma and comprises a vibrating element to be driven by air and a hose for passing vibrating air to the mouth in order to convert the generated vibration there into different sounds. Drawbacks of this prosthesis are the mediocre quality of the voice and the visual unattractiveness of the vibration source and the hose leading to the mouth, which further adversely affect the convenience of use.

A voice-producing prosthesis is known from EP-B-0 573 888. In this prosthesis a vibrations-generating element is placed in the shunt valve in the wall between the trachea and the esophagus. However, in this prosthesis the problem also occurs that the voice sounds substantially less natural and less understandable than that of a person with healthy, intact vocal cords.

U.S. patent 1,836,816 discloses a voice-producing apparatus in which a supply hose for connection to a stoma connects to a housing with a passage, and in which a discharge hose connects to the housing. The housing is provided with an opening communicating with the supply hose. Breathing takes place via this opening. By closing the opening with, e.g., the thumb, air is passed via the housing over a reed which is thereby vibrated. Sound thus generated is passed via the discharge hose into the mouth and can be converted there into speech by articulating to a certain degree.

According to the specification, the reed, when vibrating, vigorously strikes a rubber stop and thereby closes an opening via which the supply hose communicates with the discharge hose. According to this document, a sound is thereby generated which, in addition to the base frequency, contains relatively strong harmonic frequencies and thus sounds natural. Also when using this apparatus, however, it is found that there is a need for an improvement towards a natural, well understandable voice.

Document US 4 223 411 describes a voice-producing prosthesis according to the preamble of claim 1.

It is an object of the invention to provide a solution which enables patients, after a laryngectomy operation, to possess a more natural and better understandable voice than is the case when using the discussed prostheses.

This object is achieved according to the present invention by carrying out a voice-producing prosthesis according to claim 1.

By abruptly impeding movements of the vibrating element or the vibrating elements, a source sound is obtained which, during decomposition into sinusoidal frequencies in addition to a sinusoidal base frequency, contains a large number of overtones. Because this sound is generated near the position of the natural vocal cords and sound intensity and sound frequency are influenced in conjunction with each other under the influence of the speed of the airflow, a substantially improved understandability and a more natural voice are obtained.

Very advantageous practical examples of the invention are laid down in subclaims 2-6.

According to a practical possibility of the invention, a one-way valve is provided according to claim 6. By incorporating a voice-producing prosthesis according to the invention as a modular part in such a valve, it is made possible with a limited number of variants of the valve and the prosthesis to achieve a high degree of adaptation to, on the one hand, the patient's stature and, on the other hand, the patient's original vocal properties. In principle, such a voice-producing prosthesis can be used in any valve between the trachea and the esophagus.

Further objects, structural aspects and structural details of the invention will be described and explained below by means of practical examples, with reference to the drawings in which:
Fig. 1 is a schematized cross-sectional side view of a patient's head-throat area after laryngectomy and placement of a prosthesis between the trachea and the upper part of the esophagus,
Fig. 2 is a cross-sectional side view of a prosthesis for implantation in the wall between a trachea and an upper part of an esophagus with incorporated voice-producing prosthesis,
Fig. 3 is a top view of the prosthesis shown in Fig. 2, and
Fig. 4 is a side view of a series of diagrammatic representations of the vibrating element of a voice-producing prosthesis shown in Figs. 2 and 3 in successive operating conditions.

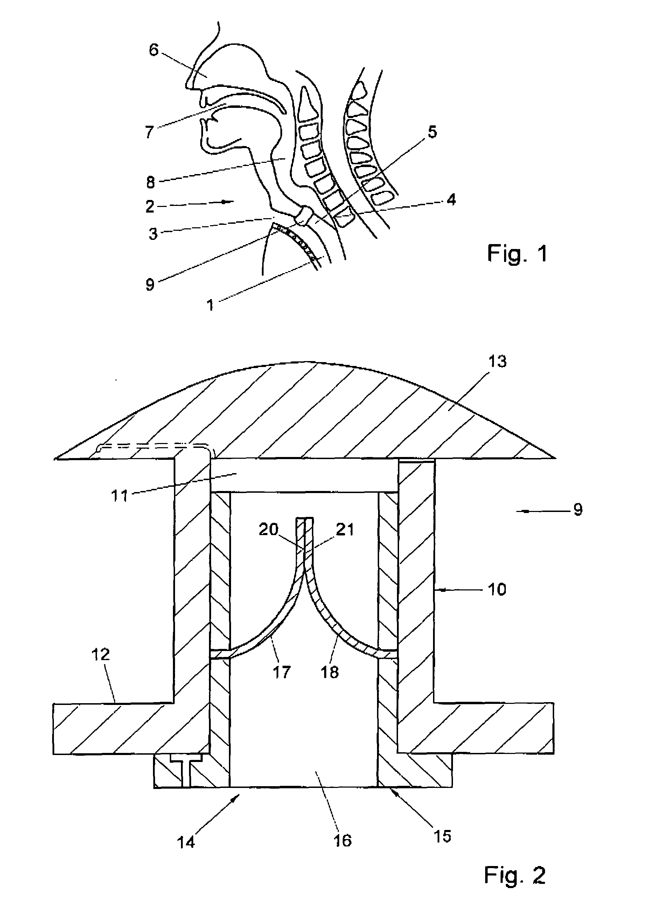

First of all, on the basis of Fig. 1, the situation will be described in which the proposed voice-producing prostheses can be used.

In a patient's head-throat portion shown in Fig. 1, the larynx, including the vocal folds and the epiglottis, has been removed as part of the treatment of cancer of the throat. The trachea 1, which is no longer closable now, is passed directly outwards into the area of the throat 2 and has an opening in the form of a tracheostoma 3. Located between an upper end of the esophagus 4 and the trachea 1 is a wall with a passage 5 in which a valve 9 has been placed so that air can be passed through the nose 6 or the mouth 7 and the former throat area 8. To achieve this, the tracheostoma 3 must be temporarily closed, e.g. with a finger or by expelling air when using a tracheostoma valve as described in Dutch patent application 9202202.

Fig. 2 shows the valve 9 in more detail. The valve 9 has a housing 10 with a passage 11, a fixing flange 12 near an end of the passage 11 and an integral closing body 13. In reaction to an excess pressure in the trachea 1 and the passage 11 communicating therewith, relative to the pressure in the esophagus 4 at the height of the valve 9, the closing body 13 turns away from this passage 11 so as to release the passage 11. In the condition of rest, the closing body 13 closes the passage 11 so that foods and drinks cannot land in the trachea 1 from the esophagus 4.

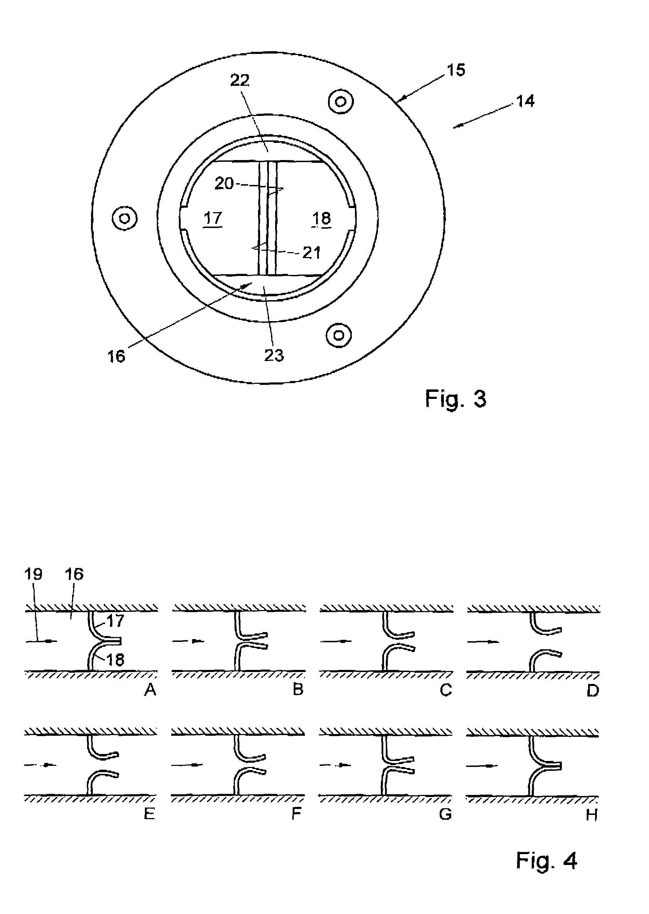

Incorporated in the valve 9 is a voice-producing prosthesis 14 which, according to this example, is composed of a housing 15, a passage 16 through the housing 15 for passing air, and two vibratingly movable vibrating elements 17, 18 for vibrating air. This prosthesis 14 is separately shown in Fig. 3.

Because the proposed voice-producing prosthesis 14 is worn near the place of the original vocal cords, the sound generated by the prosthesis 14 vibrates the air in the user's voice pipe in a natural way, and the voice thus generated can be converted by the mouth in a natural way into speech sounds. The voice-producing prosthesis is further worn inwardly so that it forms no visually disturbing element on the user's body and ensures improved convenience of use. The insertion and, if required, removal (e.g. for replacement) of the voice-producing prosthesis 14 can be carried out via the tracheostoma 3 with conventional clinical instruments.

The housing of the voice-producing prosthesis may, for that matter, also be integrated into the housing of the valve 9. Using individual housings for the valve and the voice-producing prosthesis has the advantage, however, that, on the one hand, a design may be selected for the valve for each patient on the basis of the thickness of the wall between the esophagus and the trachea and, on the other hand, independently of the external dimensions, a design may be selected for the voice-producing prosthesis of which the sound emission is most similar to the patient's original voice. Thus, in spite of a limited number of designs of the valve and the voice-producing prosthesis, a good many adaptations to different patients can be obtained.

Fig. 4 shows the vibrating elements 17, 18 in a number of successive stages A-H of a vibrating cycle. Here the vibrating elements designed as lips 17, 18 projecting from the wall of the passage 16 each form a stop for abruptly impeding the movability of the other lip 18, 17 as soon as it abuts against the relevant lip 17, 18. When air is pressed through the passage 16 at a suitable speed, the lips are opened and closed periodically as a result of a fluctuating equilibrium between aerodynamic and elastic forces which make these lips 17, 18 produce air pulses. In the condition of rest, the lips 17, 18 abut against each other with pretension.

By using lips with suitable geometry and material properties, a base frequency suitable for men and women can be obtained. By trimming the lips 17, 18, the sound produced by the prosthesis 14 can be precisely adjusted to a patient's wishes.

The lips 17, 18 each have a free end 20, 21 which, in the condition of rest, is directed downstream in a direction substantially parallel to the passage 16. This ensures that, even if the lips 17, 18 are shortened, the lips 17, 18, in the condition of rest, abut against each other with pretension. Because the shortening of the lips 17, 18 results in a decrease of the pretension at which the lips 17, 18, in the condition of rest, abut against each other, a very effective influence on the sound produced is obtained when shortening the lips 17, 18.

The patient, when speaking, can control the sound volume in a natural way by expelling air more strongly or more gently via the passageway 16. When the airflow through the passageway 16 in the direction indicated by an arrow 19 is stronger, the base frequency of the voice produced rises with the sound volume. This connection between sound volume and base frequency is also present in the voice production by means of healthy vocal cords and is experienced as natural intonation.

Because the lips are arranged to vibrate in reaction to an airflow through the passage 16, the lips are activated in a natural way to emit sound, and no separate sources of energy are required to drive the lips.

Because the lips 17, 18, during vibration, repeatedly come to abut against each other, the lips 17, 18 each form a stop against which is repeatedly struck by the other lip 18, 17 during vibration. The resulting vibration pattern therefore deviates from a sinusoidal vibration pattern and forms a composed sound built up from a sinusoidal base frequency and higher sinusoidal frequencies. These higher frequencies are important because they can be selectively amplified in the cavities of the throat, nose and mouth according to different patterns so as to produce different sounds.

The lips each form a vibrating element which projects from a substantially immovable connection to the housing 15 in the passage 16, the stop in the form of the other lip being located opposite a portion of the vibrating element spaced from this immovable connection. In principle, it is also possible to generate such a vibration pattern by using a single lip which vibrates against a fixed stop. However, by using two lips which each function as a stop for the other lip, a strong sound with a natural tone is simply obtained at a given base frequency and air displacement.

Since the portions 20, 21 of the lips 17, 18 functioning as stops are each located opposite a portion 21, 20 of the other lip 18, 17 located near a free end of the other lip 18, 17, the movement of the vibrating lips 17, 18 is very abruptly arrested during each cycle. This is advantageous for generating sound with a slight decrease of the intensity of the overtones, i.e. of which overtones have a great intensity.

For this purpose, it is further advantageous that in the condition of rest the vibrating element abuts against the stop and, more in particular, abuts against the stop with pretension.

For a proper understandability of voice, it is advantageous if the prosthesis generates air pulses of which the frequency spectrum contains a base frequency with overtones, the decrease of the intensity of the overtones being at most 18 dB/octave and preferably at most 12 dB/octave. In this connection, it is important that at most the second or the first derivative of the air pulse signal shows a discontinuity during each period.

A very good imitation of natural voice is obtained if the prosthesis is arranged to carry out displacements of which the first derivative shows discontinuities. Vibrations are then generated with a frequency spectrum of sinusoids composed of a base frequency and frequencies above, of which the intensity is lower by 10-14 dB/octave and preferably about 12 dB/octave. The fact that such a sound characteristic is advantageous for the understandability of synthetic or resynthetized speech is known per se from a publication entitled Effect of Glottal Pulse Shape on the Quality of Natural Vowels; A.E. Rosenberg in The Journal of the Acoustical Society of America; Vol. 49, No. 2, 1971.

As best shown in Fig. 3, the prosthesis 14 has passages 22, 23 along the lips 17, 18, for passing air along the lips 17, 18 in each position of the lips 17, 18. The ample lateral clearance formed by the passages 22, 23 between the lips 17, 18 and the wall of the passage 16 prevents the lips 17, 18 from sticking to the wall of the passage 16. In the figure, this passage 16 is shown in round cross-section, but it may also have another form of cross-section.

## Claims

1. A voice-producing prosthesis, comprising:
a housing (15) for placement in a passage (5) through a wall between the esophagus (4) or throat and an upper region of the trachea (1),
a passage (16) through said housing (15) for passing air, and
at least one vibratingly movable vibrating element (17, 18) for vibrating air to produce sound having at least one base frequency, and at least one stop (20, 21) for abruptly impeding the movability of the at least one vibrating element (17, 18) in at least one position, the at least one vibrating element being arranged to increase the volume and the base frequency of the sound with the intensity of an airflow through the passage
**characterized by** the stop (20, 21) being formed by a second vibrating element (17, 18), the vibrating elements (17, 18) being in the form of two mutually substantially identical flexible lips (17, 18) projecting into said housing passage (16) from a wall of the passage (16), which lips, in the condition of rest, abut against each other with free ends portions of said lips directed in a direction substantially parallel to said housing passage (16).

2. A prosthesis according to claim 1, wherein the at least one vibrating element (17, 18), in the condition of rest, abuts against said stop (20, 21) with pretension.

3. A prosthesis according to claim 1 or 2, further comprising at least one passage (22, 23) along the at least one vibrating element (17, 18) for passing air along the at least one vibrating element (17, 18) in any position of the vibrating element (17, 18).

4. A prosthesis according to any one of the preceding claims, wherein the vibrating element (17, 18) is arranged to carry out displacements, resulting in air pulses, of which the first derivative shows discontinuities.

5. A prosthesis according to any one of the preceding claims, wherein the vibrating element (17, 18) is arranged to generate vibrations with a frequency spectrum of sinusoids comprising a base frequency and frequencies above, of which the intensity is lower by 10-14 dB/octave.

6. A one-way valve for placement in a passage through a wall (5) between the esophagus (4) and the trachea (1) of a patient, comprising a housing (12) with a passage (11) and a closing body (13) for closing said passage (11), further comprising a prosthesis according to any one of the preceding claims located in the passage (11).

## Patentansprüche

1. Stimmprothese mit:
einem Gehäuse (15) zum Anordnen in einem Durchgang (5) durch eine Wand zwischen der Speiseröhre (4) oder der Kehle und einem oberen Bereich der Luftröhre (1),
einem Durchgang (16) durch das Gehäuse (15) zum Durchlassen von Luft, und
mindestens einem vibrierend bewegbaren Vibrationselement (17, 18), das Luft in Schwingungen versetzt, um Schall mit mindestens einer Grundfrequenz zu erzeugen, und mindestens einem Anschlag (20, 21) zum abrupten Hemmen der Bewegbarkeit des mindestens einen Vibrationselements (17, 18) in mindestens einer Position, wobei das mindestens eine Vibrationselement derart ausgebildet ist, dass es die Lautstärke und die Grundfrequenz des Schalls mit der Intensität einer durch den Durchgang strömenden Luftströmung erhöht,
**dadurch gekennzeichnet, dass** der Anschlag (20, 21) durch ein zweites Vibrationselement (17, 18) gebildet ist, wobei die Vibrationselemente (17, 18) in Form zweier miteinander im wesentlichen identischen flexiblen Lippen (17, 18) vorliegen, die von einer Wand des Durchgangs (16) in den Gehäusedurchgang (16) ragen, wobei die Lippen im Ruhezustand aneinander anliegen, wobei die freien Endbereiche der Lippen im wesentlichen parallel zu dem Gehäusedurchgang (16) gerichtet sind.

2. Prothese nach Anspruch 1, bei der das mindestens eine Vibrationselement (17, 18) im Ruhezustand an dem Anschlag (20, 21) unter Vorspannung anliegt.

3. Prothese nach Anspruch 1 oder 2, ferner mit mindestens einem Durchgang (22, 23) entlang dem mindestens einen Vibrationselement (17, 18) zum Leiten von Luft entlang dem mindestens einen Vibrationselement (17, 18) in jeglicher Position des Vibrationselements (17, 18).

4. Prothese nach einem der vorhergehenden Ansprüche, bei der das Vibrationselement (17,18) zum Durchführen von Verschiebungen ausgebildet ist, die in Luftimpulsen resultieren, deren erste Ableitung Diskontinuitäten zeigt.

5. Prothese nach einem der vorhergehenden Ansprüche, bei der das Vibrationselement (17, 18) zum Erzeugen von Vibrationen mit einem Frequenzspektrum von Sinuswellen ausgebildet ist, die eine Grundfrequenz und über dieser liegende Frequenzen umfassen, deren Intensität um 10-14 dB/Oktave niedriger ist.

6. Einwegventil zum Anordnen in einem Durchgang (5) durch eine Wand zwischen der Speiseröhre (4) und der Luftröhre (1) eines Patienten, mit einem einen Durchgang (11) aufweisenden Gehäuse (12) und einem Schließkörper (13) zum Schließen des Durchgangs (11), ferner mit einer in dem Durchgang (11) angeordneten Prothese nach einem der vorhergehenden Ansprüche.

## Revendications

1. Prothèse vocale comprenant :
un logement (15) destiné à être placé dans un passage (5) à travers une paroi entre l'oesophage (4) ou la gorge et une région supérieure de la trachée (1),
un passage (16) à travers ledit logement (15) destiné au passage de l'air, et
au moins un élément vibrant mobile par vibrations (17, 18) pour faire vibrer l'air de manière à produire un son ayant au moins une fréquence de base, et au moins une butée (20, 21) pour empêcher brusquement la mobilité d'au moins un élément vibrant (17, 18) dans au moins une position, au moins un élément vibrant étant agencé pour augmenter le volume et la fréquence de base du son avec l'intensité d'un écoulement d'air à travers le passage,
**caractérisée en ce que** la butée (20, 21) est formée par un second élément vibrant (17, 18), les éléments vibrants (17, 18) étant sous la forme de deux lèvres flexibles sensiblement identiques (17, 18) faisant saillie dans ledit passage du logement (16) depuis une paroi du passage (16), lesdites lèvres, à l'état de repos, venant en butée l'une contre l'autre avec les parties d'extrémités libres desdites lèvres dirigées dans une direction sensiblement parallèle au passage du logement (16).

2. Prothèse selon la revendication 1, dans laquelle au moins un élément vibrant (17, 18), à l'état de repos, vient en butée contre ladite butée (20, 21) avec une certaine prétension.

3. Prothèse selon la revendication 1 ou 2, comprenant en outre au moins un passage (22, 23) le long d'au moins un élément vibrant (17, 18) pour faire passer de l'air le long d'au moins un élément vibrant (17, 18) dans toute position de l'élément vibrant (17, 18).

4. Prothèse selon l'une quelconque des revendications précédentes, dans laquelle l'élément vibrant (17, 18) est agencé pour effectuer des déplacements, entraînant des impulsions d'air, dont le premier dérivé montre des discontinuités.

5. Prothèse selon l'une quelconque des revendications précédentes, dans laquelle l'élément vibrant (17, 18) est agencé de manière à générer des vibrations avec un spectre de fréquences de sinusoïdes comprenant une fréquence de base et des fréquences supérieures, dont l'intensité est inférieure de 10 à 14 dB/octave.

6. valve anti-retour destinée à être placée dans un passage à travers une paroi (5) entre l'oesophage (4) et la trachée (1) d'un patient, comprenant un logement (12) avec un passage (11) et un corps de fermeture (13) pour fermer ledit passage (11), comprenant en outre une prothèse selon l'une quelconque des revendications précédentes située dans le passage (11).
